# EUROPEAN PATENT SPECIFICATION

(11) **EP 3 883 920 B1**
(45) Date of publication and mention of the grant of the patent: **05.03.2025**
(21) Application number: 19808739.7
(22) Date of filing: 19.11.2019
(51) Int. Cl.: C07C 255/58, C07C 269/00, C07C 271/22, C07C 271/28, C07C 271/34, C07C 273/18, C07C 333/08, C07D 207/00, C07J 1/00

(54) **N-TRIFLUORMETHYLCARBONYL COMPOUNDS AND METHODS FOR THEIR SYNTHESIS**
N-TRIFLUORMETHYLCARBONYLVERBINDUNGEN UND VERFAHREN ZU IHRER SYNTHESE
COMPOSÉS N-TRIFLUORMÉTHYLCARBONYLE ET LEURS PROCÉDÉS DE SYNTHÈSE

(30) Priority: 23.11.2018 DE 102018129597
(43) Date of publication of application: 29.09.2021
(73) Proprietor: Rheinisch-Westfälische Technische Hochschule (RWTH) Aachen, 52056 Aachen (DE)
(72) Inventor: SCHOENEBECK, Franziska, 50678 Köln (DE); SCATTOLIN, Thomas, 86130 St. Georges les Baillargeaux (FR)
(74) Representative: Michalski Hüttermann & Partner Patentanwälte mbB
(86) International application number: PCT/EP2019/081850
(87) International publication number: WO 2020/104484

(56) References cited:
- WO-A1-2017/110865
- AKIRA SEKIYA ET AL: "The reaction of 2-trifluoromethyl-3,3-difluorooxaziridine with nucleophiles.", JOURNAL OF FLUORINE CHEMISTRY, vol. 14, no. 4, 1 October 1979 (1979-10-01), NL, pages 289 - 297, XP055659997, ISSN: 0022-1139, DOI: 10.1016/S0022-1139(00)82973-5
- SUNDERMEYER W ET AL: "Perfluoralkyl- und fluorcarbonyl-hydrazine", JOURNAL OF FLUORINE CHEMISTRY, ELSEVIER, NL, vol. 34, no. 2, 1 December 1986 (1986-12-01), pages 251 - 254, XP026730936, ISSN: 0022-1139, [retrieved on 19861201], DOI: 10.1016/S0022-1139(00)85075-7
- GONTAR A F ET AL: "TRIFLUOROMETHYLCARBAMATES", MENDELEEV CHEMISTRY JOURNAL - VSESOYUZNOE KHIMICHESKOE OBSHCHESTVO IM. D.I. MEDEELEVA. ZHURNAL, ALLERTON PRESS, NEW YORK, NY, US, vol. 22, 1 January 1977 (1977-01-01), pages 119 - 121, XP009518314, ISSN: 0025-925X
- A. D. GONTAR ET AL: "Trifluoromethylfluorocarbonyl azanion", BULLETIN OF THE ACADEMY OF SCIENCES OF THE USSR, DIVISION OF CHEMICAL SCIENCES., vol. 25, no. 1, 1 January 1976 (1976-01-01), US, pages 202 - 204, XP055659966, ISSN: 0568-5230, DOI: 10.1007/BF00925656
- MARIUS MAMONE ET AL: "Electrophilic Amination of Fluoroalkyl Groups on Azodicarboxylate Derivatives", JOURNAL OF ORGANIC CHEMISTRY, vol. 80, no. 3, 16 January 2015 (2015-01-16), US, pages 1964 - 1971, XP055659732, ISSN: 0022-3263, DOI: 10.1021/jo502638y
- JOHN A. YOUNG ET AL: "Fluorocarbon Nitrogen Compounds. I. Perfluorocarbamic Acid Derivatives, Amides and Oxazolidines 1", JOURNAL OF THE AMERICAN CHEMICAL SOCIETY, vol. 78, no. 21, 1 November 1956 (1956-11-01), pages 5637 - 5639, XP055659721, ISSN: 0002-7863, DOI: 10.1021/ja01602a046
- WILLIAM A. SHEPPARD: "N-Fluoroalkylamines. I. Difluoromethines", JOURNAL OF THE AMERICAN CHEMICAL SOCIETY, 5 October 1965 (1965-10-05), pages 4338 - 4341, XP055660143, Retrieved from the Internet <URL:https://pubs.acs.org/doi/pdf/10.1021/ja00947a021> [retrieved on 20200121]
- YAGUPOL'SKII L M ET AL: "NEW ACYLATING AGENTS CONTAINING THE N,N-BIS(TRIFLUOROMETHYL)AMINO GROUP", JOURNAL OF ORGANIC CHEMISTRY USSR, PLENUM PUBL. CORP, US, vol. 18, 1 January 1982 (1982-01-01), pages 1958 - 1959, XP009518313, ISSN: 0022-3271
- MOTORNYI ET AL: "NEW METHOD FOR THE PRODUCTION OF OF N-PERFLUOROALKYL-N-METHYLCARBAMATES", JOURNAL OF GENERAL CHEMISTRY USSR, CONSULTANTS BUREAU, NEW YORK, NY, US, vol. 33, 1 January 1963 (1963-01-01), pages 3319 - 3320, XP009518299, ISSN: 0022-1279

## Description

The present invention relates to the field of N-trifluoromethyl compounds, especially *N-*trifluoromethylcarbonyl compounds.

The amide motif is of central importance, impacting all sciences, life, medicine and modern society. It is a preferred building block of nature and a key unit that controls stability, 3D-structure and hence function of proteins or peptides, and consequently also biological processes or related applications, such as catalysis.

The closely related compound families, e.g. carbamates and ureas, are similarly influential, finding applications as insecticides, in polymers (foams, elastomers, polyurethanes), as preservatives, cosmetics and in medical treatment as, for example, chemotherapy and HIV agents.

Fluorinated compounds play an eminent role, especially but not limited to the pharmaceutical field due to the steric similarity but different chemical/physical behaviour between fluorine and hydrogen and thus methods for the specific synthesis of fluorinated compounds are subject of immense research.

William A. Sheppard, "N-Fluoroalkylamines. I. Difluoromethines", Journal of the American Chemical Society, 5 October 1965, pages 4338-4341, describes the reaction of isothiocyanates providing a new general route to difluoroazomethines and being particularly useful for preparing N-aryldifluoroazomethines which are trapped as N-(trifluoromethyl)-N-(fluoroformyl)anilines by carbonyl fluoride addition.

Due to the importance of amides and related compounds, there is a constant need for methods for the synthesis of fluorinated amides and related compounds, especially compounds which comprise an N-CF₃ motif. Additionally, there is a significant demand for novel compounds having such an N-CF₃ motif.

This object is met by the method of Claim 1 of the present invention. Accordingly, a method for the synthesis of N-trifluoromethylcarbonyl compounds having the structure R-N(CF₃)CO-LG, whereby R is an organic moiety from isothiocyanates R-NCS and LG is a leaving group is provided, comprising the step a)
a) contacting the isothiocyanate R-NCS with AgF and an electrophilic C₁-compound where the carbon is in the formal oxidation state (+IV)

Surprisingly, it has been found that by doing so for many isothiocyanates a conversion into *N-*trifluoromethylcarbonyl compounds can be observed. For most applications within the present invention, at least one of the following advantages could be observed:
- The reaction usually proceeds smoothly and straightforwardly.
- The reaction can be performed at room temperature or slightly elevated temperatures.
- The reaction avoids toxic reactants such as HgF₂ which have been used before.
- A wide range of isothiocyanate reactants can be used, including reactants which have a complex structure, which is usually not affected in the course of the reaction.
- Isothiocyanates are substances which are easily obtainable, thus the reaction can be used handily in many fields of organic chemistry.
- The reaction usually occurs with retention of configuration.
- A wide variety of functional groups are tolerated.

The term "organic moiety" especially means and/or includes a structure whereby a carbon atom is adjacent to the N(CF₃)CO-unit and bound to that unit with a single bond.

The term "leaving group" especially means and/or includes any chemical moiety which is able to depart with a pair of electrons in heterolytic bond cleavage. Especially preferred leaving groups are halogens, here especially preferred fluoride and/or chloride, sulfonic esters, especially tosylates, heterocyclic compounds, especially imidazoles. Especially preferred are fluoride and chloride.

The term "electrophilic C₁-compound" especially means and/or includes a compound of the chemical structure LG¹-CO-LG² whereby LG¹ and LG² are independently from each other leaving groups, including leaving groups as above. However, also preferred in this context are leaving groups of the structure -OCHal₃, whereby Hal is halogen, especially fluoride and/or chloride. The resulting compounds, e.g. Hal₃C-O(CO)O-CHal₃ have the advantage that they in the course of the reaction will form two compounds Hal-CO-Hal (as known e.g. from triphosgene, the compound with Hal=Cl) which themselves are electrophilic C₁-compounds. Similarly a compound where only LG is -OCHal₃ will form additionally (only) one compound Hal-CO-Hal (e.g. known from diphosgene, Cl-CO-OCl₃).

AgF can be used either directly or in its components, i.e. via a cationic silver component (e.g. AgNO₃, AgOTf) and nucleophilic fluoride (e.g. KF, NaF).

It should be noted that the isothiocyanate R-NCS and/or the electrophilic C₁-compound and/ or the AgF may be formed *in situ* during the course of the reaction. Therefore it is a preferred embodiment of the present invention that the method comprises another step a0) which is performed before step a)

### a0) Formation of the isothiocyanate R-NCS and/or the electrophilic C₁-compound and/or the AgF

According to a preferred embodiment of the present invention, AgOCF₃ is used to directly transform R-NCS to N-CF₃ carbamoyl fluorides. The AgOCF₃ generates AgF and COF₂ in *situ.*

Other OCF₃-containing compounds can also be used as COF₂ source.

It is especially advantageous that the molar ratio of AgOCF₃ to isothiocyanate is ≥1.0, preferably ≥ 1.1 to ≤ 3, more preferred ≥ 1.5 to ≤ 2,5, most preferred ≥ 1.8 to ≤ 2.

The AgOCF₃ itself can be provided in substance or itself be generated *in situ* (either in a separate step or during step a0).

According to a further preferred embodiment of the present invention, AgF is formed *in situ* by cross-reaction of a silver salt and a nucleophilic fluoride compound, whereby suitable silver salt precursors include AgOTf, AgNO₃, Ag halides (other than fluoride) and nucleophilic fluoride compounds include tetraalkylammonium fluorides and alkali fluorides. The fluoride can also be formed *in situ* from an organic compound that contains fluoride by suitable chemical transformation, e.g. by a nucleophilic aromatic substitution reaction of a suitable aryl fluoride with a nucleophile or by displacement from alternative fluorine containing compounds, such as, for example R-SO₂-F.

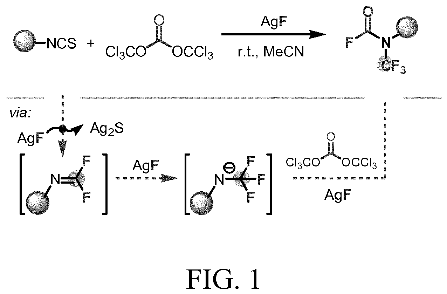

Without being bound to any chemical theory, the inventors believe that when an excess of AgF is used (cf. the further description) then in most applications of the present invention a conversion of the isothiocyanate to an N-trifluoromethylcarbamoyl fluoride occurs according to the mechanism shown in Fig. 1 of this application for a special embodiment of the present invention:
Accordingly, the sulfur of the isothiocyanate is replaced by two fluorides via an exchange reaction with the AgF, which subsequently reacts to Ag₂S. Thus a compound of the structure R-N=CF₂ is formed. Additional fluoride forms the CF₃-unit following a nucleophilic attack of the nitrogen to the carbon of the electrophilic C₁-compound. If fluoride is used in excess, then another substitution leads to the N-trifluoromethylcarbamoyl fluoride.

According to a preferred embodiment of the present invention, in step a) the molar ratio of effective electrophilic C₁ compound to isothiocyanate is ≥1.0 to ≤ 2, preferably ≥ 1.2 to ≤ 1.5. The term "effective" means that if in the course of the reaction the additional electrophilic C₁ compound is formed then this is taken into account, e.g. when triphosgene is used as electrophilic C₁ compound then the amount of effective electrophilic C₁ compound is three times the amount of triphosgene. When diphosgene Cl-CO-OCl₃ is used, the effective electrophilic C₁ compound is twice the amount of diphosgene.

According to a preferred embodiment step a) is carried out in the presence of one or more additional fluoride compounds, which are preferably used in a molar ratio of ≥1 (compared to the isothiocyanate). Preferred fluorine compounds are alkali and earth alkali fluorides, especially LiF, NaF, KF, RbF, CsF, CaF₂, MgF₂, fluorides from quaternary amines, especially TMAF and TBAF (NMe₄F, NBu₄F) and other metal fluorides, especially CuF₂. Preferably the fluorine compound is used in a molar ratio of this fluorine compound to the isothiocyanate in ≥1, still preferred ≥2 and most preferred ≥3 to ≤ 4.

According to a preferred embodiment of the present invention, in step a) the molar ratio of AgF to isothiocyanate in step a) is ≥4, preferably ≥ 5 to ≤ 8. When doing so usually the *N-*trifluoromethylcarbamoyl fluoride is formed, as described above. In many cases the yields are so high that AgF can be used as sole fluorine compound which is therefore an embodiment of the present invention.

However, according to an alternative preferred embodiment of the present invention, in step a) the molar ratio of AgF to isothiocyanate is ≥3 to ≤ 4. When doing so then - in case no further fluoride compound is present, which is, however, a preferred embodiment of the invention - in many applications in the product R-N(CF₃)CO-LG the moiety LG will be the LG of the electrophilic C₁-compound or a mixture of this compound with *N-*trifluoromethylcarbamoyl fluoride can be observed as reaction products. It goes without saying that in case the leaving group of the electrophilic C₁-compound is -OCHal₃ then of course LG will be Hal.

Yet another to an alternative preferred embodiment of the present invention in step a) the molar ratio of AgF to isothiocyanate is ≥2 and another fluorine compound is used in a molar ratio of ≥1 (compared to the isothiocyanate). Also here, depending on the fluoride concentration, in many applications in the product R-N(CF₃)CO-LG the moiety LG will be the LG of the electrophilic C₁-compound or a mixture of this compound with *N-*trifluoromethylcarbamoyl fluoride can be observed as reaction products.

Preferably step a) is carried out in an organic nonpolar or dipolar aprotic solvent. Especially preferred are nitrile-based solvents, especially acetonitrile, benzonitrile, propionitrile, ether-based solvents, especially glyme, diglyme, triglyme, MeTHF, Et₂O, THF, dioxane, NMP, DCM, toluene, THF with acetonitrile being most preferred.

Preferably step a) is carried out at a temperature of ≥ 0°C and ≤ 70°C, more preferred ≥ 20°C and ≤ 30°C. In case that AgOCF₃ is used as an AgF and/or COF₂ precursor, preferably the method is carried out at a temperature of ≥ 30°C and ≤ 70°C, more preferred ≥ 40°C and ≤ 50°C.

The object of the present invention is furthermore met by the method of Claim 8 of the present invention. Accordingly a method for the synthesis of N-trifluoromethyl amides is provided comprising the step of contacting a compound R-N(CF₃)CO-LG, with R and LG as defined above, with a organometallic compound.

Surprisingly it has been shown that by doing so, N-trifluoromethyl amides can be obtained in excellent yields for many applications. For most applications within the present invention, at least one of the following advantages could be observed:
- The reaction usually proceeds smoothly and straightforwardly.
- The reaction can be performed at room temperature or slightly elevated temperatures.
- The reaction usually occurs with retention of configuration.
- The reaction can occur without temperature control.
- Reaction can occur without the need for careful reaction control [addition of the reactant].

Preferred metal-organic compounds are organomagnesium compounds (Grignard Reagents), organolithium compounds and organozinc compounds.

Preferably the method is carried out in an organic nonpolar or dipolar aprotic solvent. Especially preferred are DCM, Et₂O, toluene, THF with toluene being most preferred.

Preferably the method is carried out at a temperature of ≥ -100°C and ≤ 25°C, more preferred ≥ 0°C and ≤ 25°C.

The object of the present invention is furthermore met by the method of claim 10 of the present invention. Accordingly a method for the synthesis of *N*-trifluoromethyl ureas, *N-*trifluoromethyl carbamates, N-trifluoromethyl thiocarbamates and/or *N*-trifluoromethyl selenocarbamates is provided, comprising the step of reacting a compound R-N(CF₃)CO-LG, whereby R is an organic moiety and LG is a leaving group, with an amine, alcohol, thiol and selenol, whereby the compound R-N(CF₃)CO-LG is prepared as described above.

Surprisingly it has been shown that by doing so these compounds can be obtained in excellent yields for many applications. For most applications within the present invention, at least one of the following advantages could be observed:
- The reaction usually proceeds smoothly and straightforwardly.
- The reaction can be performed at room temperature or slightly elevated temperatures.
- The reaction usually occurs with retention of configuration.
- The reaction can occur with the neutral nucleophile or the deprotonated nucleophile.
- The reaction proceeds in a highly selective manner.

N-trifluoromethyl carbamates and/or N-trifluoromethyl thiocarbamates which are both very suitable compounds, especially in the pharma sector, are so far unknown, same as selenocarbamates derivatives. Many of them have, however, shown to have a greater stability than their N-methyl-counterparts. Therefore, the present invention also relates to compounds of the structure R-N(CF₃)-CO-X-R' with R and R' being organic moieties and X being S, O or Se.

Preferably the method is carried out in an organic nonpolar or dipolar aprotic solvent. Especially preferred are DCM, Et₂O, toluene, THF with DCM and THF being most preferred.

Preferably the method is carried out at a temperature of ≥ 0°C and ≤ 50°C, more preferred ≥ 20°C and ≤ 30°C.

Preferably the method is carried out using a base in an amount of ≥ 1 and ≤ 5 equivalents, relative to the reactant R-N(CF₃)CO-LG. Preferred bases are inorganic bases such as, carbonate, bicarbonate and phosphate, such as cesium carbonate, sodium bicarbonate and potassium phosphate, tertiary amines, especially trialkylamines such as triethylamine, Hünig's base (*N,N*-diisopropylethylamine), N-methylmorpholine, pyridine, 1,8-diazabicyclo[5.4.0]undec-7-ene (DBU), 1,5-diazabicyclo[4.3.0]non-5-ene (DBN), 1,4-diazabicyclo[2.2.2]octane (DABCO) or mixtures thereof.

Preferably the method is carried out using ≥ 0.1 and ≤ 0.5 equivalents (relative to the reactant R-N(CF₃)CO-LG) of a nucleophilic catalyst selected out of the group comprising pyridine-based catalysts, especially pyridine and DMAP and five-membered *N*-heterocyclic compounds, especially imidazole. This has shown to furthermore increase yields in many embodiments of the invention.

It goes without saying that in the two methods described before the reactant R-N(CF₃)CO-LG is preferably synthesized as described before, which is insofar a preferred embodiment of the invention. Therefore the present invention relates to a method comprising the steps:
a) contacting an isothiocyanate R-NCS with AgF and an electrophilic C₁-compound where the carbon is in the formal oxidation state (+IV) to obtain a compound R-N(CF₃)CO-LG
b) contacting a compound R-N(CF₃)CO-LG with an organometallic compound, amine, alcohol, thiol and/or selenol.

The aforementioned components, as well as the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the sub-claims and the following description of the respective examples -which in an exemplary fashion--show preferred embodiments according to the invention.

### General procedure for the synthesis of N-trifluoromethylcarbamoyl fluorides:

A 20 mL vial was charged with the isothiocyanate (2 mmol, 1 equiv.), silver fluoride (10 mmol, 5 equiv.) and triphosgene (237 mg, 0.8 mmol, 0.4 equiv.). Acetonitrile (10 mL) was added quickly and the vial was sealed (if the isothiocyanate was a liquid or an oil it was added as a solution in the solvent). The mixture was stirred at room temperature. Afterwards the crude mixture was added at once to Et₂O (40 mL) and stirred for 10 minutes. The solid was filtered through celite and the solvents were then evaporated. The obtained crude material was dissolved in Et₂O and filtered on a pad of celite again to remove the last traces of salt byproducts. The N-trifluoromethylcarbamoyl fluoride was then obtained in a technical grade purity ranging from 90 to 99%.

Using this general procedure, the following compounds 1 to 30 were synthesized:

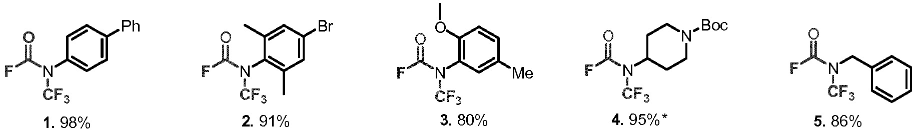

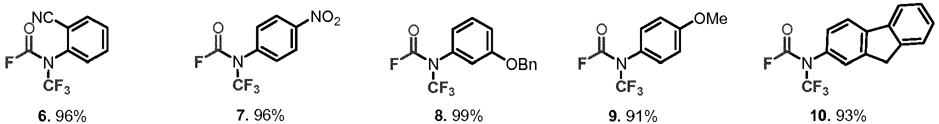

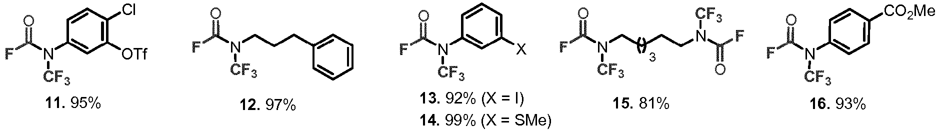

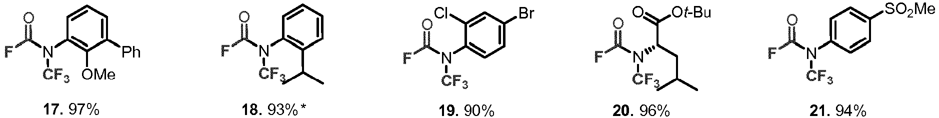

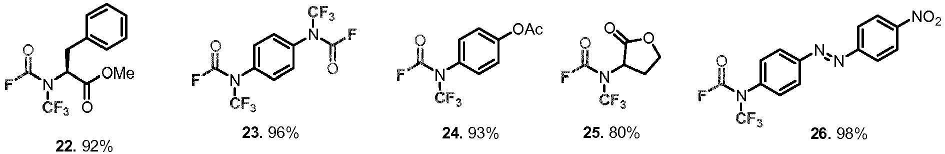

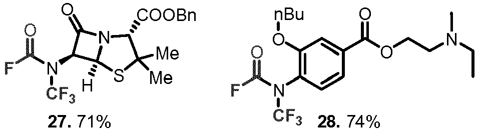

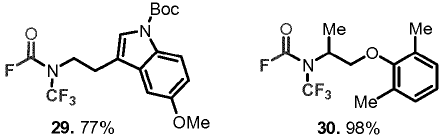

Exemplarily the synthesis of compound 1 is described in more detail:
**[1,1'-Biphenyl]-4-yl-*N*-trifluoromethylcarbamoyl fluoride 1** : The title compound was obtained as a white solid after 15 h in 98% yield (555 mg) using 4-isothiocyanato-1,1'-biphenyl (423 mg) following the general procedure for *N*-trifluoromethylcarbamoyl fluorides. M.p.: 94-96 °C. ¹H NMR (400 MHz, CDCl₃) δ 7.71 - 7.64 (m, 2H), 7.61 - 7.54 (m, 2H), 7.50 - 7.43 (m, 2H), 7.43 - 7.34 (m, 3H). ¹⁹F NMR (376 MHz, CDCl₃) δ -2.6 (brs, 1F), -56.2 (brs, 3F). ¹³C NMR (151 MHz, CDCl₃) δ 143.6, 142.1 (d, *J* = 300.8 Hz), 139.4, 132.0, 129.0, 128.6, 128.6, 128.2, 127.3, 119.2 (q, *J =* 264.8 Hz). MS (70eV, EI): m/z (%): 283 (100) [M+], 172 (19), 69 (5). HRMS (EI) calculated for C₁₄H₉ONF₃: 264.06308 [M-F]⁺, Found: 264.06302.

### General procedure for the synthesis of amides:

A 4 mL vial was charged with the *N*-trifluoromethylcarbamoyl fluoride (0.2 mmol, 1 equiv.), toluene (1.5 mL) and placed into a water bath at room temperature. The corresponding Grignard reagent (0.24 mmol, 1.2 equiv.) was subsequently added to the solution. The reaction mixture was stirred for 10 minutes at room temperature. Saturated aqueous ammonium chloride solution (1.5 mL) was then added. The phases were separated and the aqueous phase was further extracted with EtOAc (2x), dried over MgSO₄ and concentrated under reduced pressure. The obtained crude material was then purified by column chromatography.

Using this general procedure the compounds 31 to 44 were synthesized:

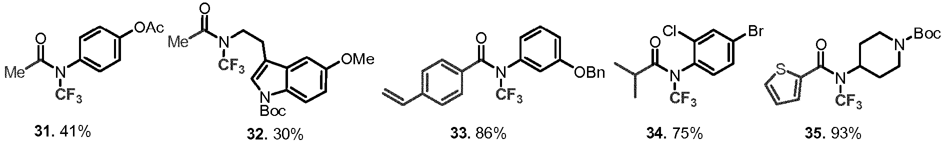

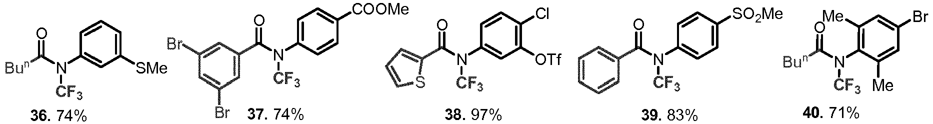

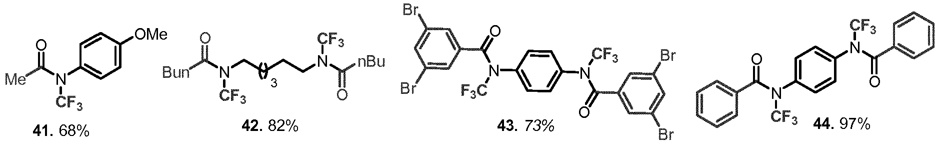

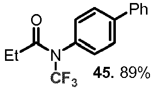

Exemplarily the synthesis of compound 38 is described in detail:
**2-Chloro-5-(N-(trifluoromethyl) thiophene-2-carboxamido)phenyl trifluoromethanesulfonate 38** : The title compound was obtained as a colorless oil in 97% yield (88 mg) using 2-chloro-5-((fluorocarbonyl)(trifluoromethyl)amino)phenyl trifluoromethanesulfonate **11** (78 mg) with 2-thienylmagnesium bromide (240 µL, 1.0M in THF) after column chromatography on silica gel with EtOAc/hexane (5/95, R_{f} = 0.20). ¹H NMR (400 MHz, CDCl₃) δ 7.60 (dd, *J =* 8.6, 1.0 Hz, 1H), 7.50 (dd, *J* = 5.0, 1.0 Hz, 1H), 7.42 (d, *J* = 2.4 Hz, 1H), 7.35 (dd, *J =* 8.6, 2.4 Hz, 1H), 7.25 - 7.23 (m, 1H), 6.92 (ddd, *J =* 5.0, 3.9, 1.0 Hz, 1H). ¹⁹F NMR (376 MHz, CDCl₃) δ -56.5 (s, 3F), -73.2 (s, 3F). ¹³C NMR (151 MHz, CDCl₃) δ 161.5, 145.8, 135.5, 135.3, 134.7, 134.1, 132.1, 131.2, 129.9, 127.5, 125.7, 119.8 (q, *J* = 265.8 Hz), 118.5 (q, *J* = 320.9 Hz). HRMS (ESI) calculated for C₁₃H₆O₄N³⁵ClF₆Na³²S₂: 475.92232 [M+Na]⁺, Found: 475.92151.

### General procedure for the synthesis of ureas:

A 4 mL vial was charged with the *N*-trifluoromethylcarbamoyl fluoride (0.2 mmol, 1 equiv.) and CH₂Cl₂ (1.5 mL). The corresponding amine (0.24 mmol, 1.2 equiv.), Hunig's base (42 µL, 0.24 mmol, 1.2 equiv.) and DMAP (2 mg, 0.02 mmol, 0.1 equiv.) were subsequently added to the solution. The reaction mixture was stirred for 15 h at room temperature. Hexane (1 mL) was then added and the reaction mixture was filtered through a pad of celite before evaporation. The obtained crude material was then purified by column chromatography.

### Stability investigation of compound 45

To challenge the stability of *N*-CF₃ amides, we subjected amide **45** to a solution of 0.5 M HCl and also 0.5 M NaOH at 50°C for 15 h (in 50:50 THF/H₂O) and observed essentially no decomposition, whereas the corresponding N-Me and N-H analogues showed decomposition. This suggests that many *N*-trifluoromethyl amides are more stable than their 'conventional' amide counterparts.

### General procedure for the synthesis of carbamates:

A 4 mL vial was charged with the N-trifluoromethylcarbamoyl fluoride (0.2 mmol, 1 equiv.) and CH₂Cl₂ (1.5 mL). The corresponding alcohol (0.24 mmol, 1.2 equiv.), Hunig's base (42 µL, 0.24 mmol, 1.2 equiv.) and DMAP (2 mg, 0.02 mmol, 0.1 equiv.) were subsequently added to the solution. The reaction mixture was stirred for 15 h at room temperature. Hexane (1 mL) was then added and the reaction mixture was filtered through a pad of celite before evaporation. The obtained crude material was then purified by column chromatography.

### General procedure for the synthesis of thiocarbamates and selenocarbamates:

A 4 mL vial was charged with the N-trifluoromethylcarbamoyl fluoride (0.2 mmol, 1 equiv.) and THF (1.5 mL). The corresponding sodium thiolate or selenolate (0.24 mmol, 1.2 equiv.) was subsequently added to the solution. The reaction mixture was stirred for 1 h at room temperature. Hexane (1 mL) was then added and the reaction mixture was filtered through a pad of celite before evaporation. The obtained crude material was then purified by column chromatography.

Using the above general methods, the following compounds 46 to 74 were synthesized:

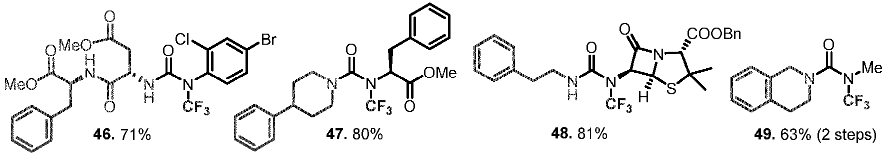

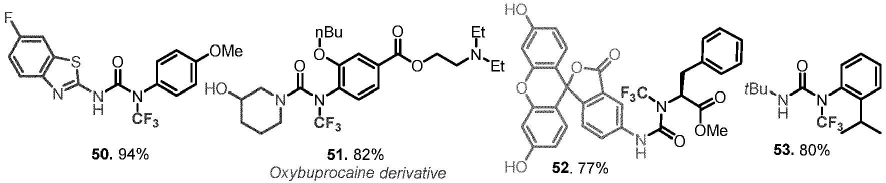

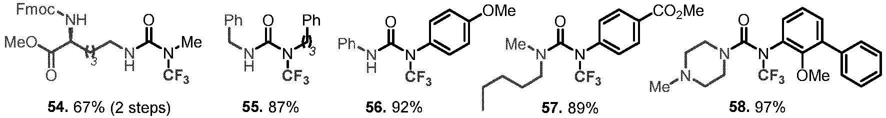

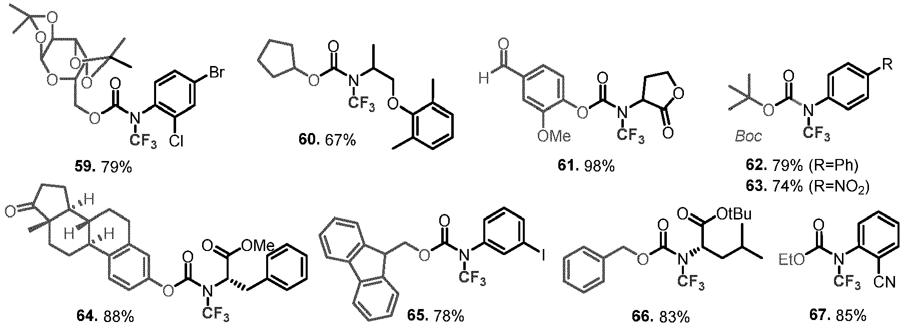

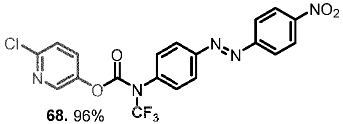

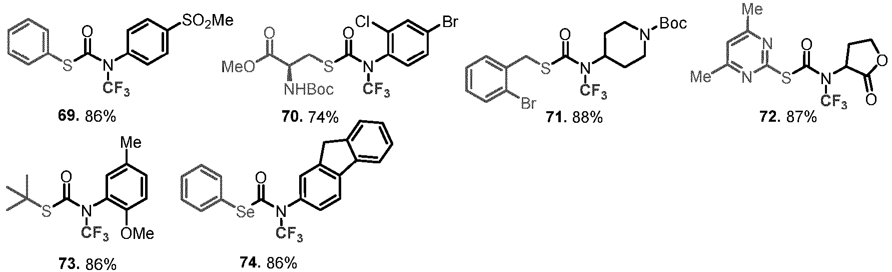

Exemplarily the synthesis of compound 46 is described in greater detail:
**Methyl (*S*)-3-(3-(4-bromo-2-chlorophenyl) -3-(trifluoromethyl)ureido)-4-(((S)-1-methoxy-1-oxo-3-phenylpropan-2-yl)amino)-4-oxobutanoate 46** : The title compound was obtained as a colorless oil in 71% yield (87 mg) using (4-bromo-2-chlorophenyl)-*N-*trifluoromethylcarbamoyl fluoride **19** (64 mg) with methyl (*S*)-3-amino-4-(((*S*)-1-methoxy-1-oxo-3-phenylpropan-2-yl)amino)-4-oxobutanoate hydrochloride [aspartame-OMe.HCl] (83 mg) in presence of *N,N* diisopropylethylamine [Hunig's base] (77 µL, 0.44 mmol, 2.2 equiv.) after column chromatography on silica gel with EtOAc/hexane (40/60, R_{f} = 0.38). [α]_{D} = +40.2 (c = 0.600 , CHCl₃, 26°C). ¹H NMR (400 MHz, CDCl₃) δ 7.69 (d, *J =* 2.2 Hz, 1H), 7.48 (d, *J =* 8.5 Hz, 1H), 7.29 - 7.25 (m, 3H), 7.17 (d, *J =* 8.5 Hz, 1H), 7.07 (dd, *J =* 6.6, 2.9 Hz, 2H), 6.93 (d, *J =* 7.6 Hz, 1H), 6.04 (s, 1H), 4.80 - 4.59 (m, 2H), 3.69 (s, 3H), 3.62 (s, 3H), 3.10 (dd, *J* = 14.0, 5.3 Hz, 1H), 3.04 - 2.95 (m, 2H), 2.55 (dd, *J* = 17.3, 7.0 Hz, 1H). ¹⁹F NMR (376 MHz, CDCl₃) δ -53.6. ¹³C NMR (151 MHz, CDCl₃) δ 172.6, 171.2, 169.5, 151.4, 136.0, 135.5, 133.9, 132.7, 132.0, 130.9, 129.2, 128.6, 127.1, 125.1, 119.6 (q, *J =* 262.5 Hz), 53.7, 52.4, 52.2, 49.9, 37.5, 35.5. HRMS (ESI) calculated for C₂₃H₂₂⁷⁹Br³⁵ClF₃N₃NaO₆: 630.02248 [M+Na]⁺, Found: 630.02124.

The particular combinations of elements and features in the above detailed embodiments are exemplary only. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims. Furthermore, reference signs used in the description and claims do not limit the scope of the invention as claimed.

## Claims

1. A method for the synthesis of *N*-trifluoromethylcarbonyl compounds having the structure R-N(CF₃)CO-LG, whereby R is an organic moiety and LG is a leaving group, from isothiocyanates R-NCS, comprising the step of
a) contacting the isothiocyanate R-NCS with AgF and an electrophilic C₁-compound where the carbon is in the formal oxidation state (+IV).

2. The method according to Claim 1, whereby the electrophilic C₁-compound comprises a compound of the chemical structure LG¹-CO-LG² whereby LG¹ and LG² are independently from each other leaving groups.

3. The method according to Claim 1 or 2, whereby the method is carried out in the presence of one or more additional fluoride compounds

4. The method according to any of the claims 1 to 3, whereby the molar ratio of AgF to isothiocyanate is ≥4.

5. The method according to any of the claims 1 to 4, whereby the molar ratio of effective electrophilic C₁ compound to isothiocyanate is ≥1.0 to ≤ 2

6. The method according to any of the claims 1 to 5, whereby the isothiocyanate R-NCS and/or the electrophilic C₁-compound and/ or the AgF are formed *in situ* during the course of the reaction.

7. The method according to any of the claims 1 to 6, whereby AgOCF₃ is used as a precursor for AgF and the electrophilic C₁-compound.

8. A method for the synthesis of *N*-trifluoromethyl amides comprising the step of contacting a compound R-N(CF₃)CO-LG, whereby R is an organic moiety and LG is a leaving group, with an organometallic compound.

9. The method according to claim 8, whereby the organometallic compound is an organomagnesium compound, organolithium compound and /or an organozinc compound.

10. A method for the synthesis of *N*-trifluoromethyl ureas, *N*-trifluoromethyl carbamates, *N*-trifluoromethyl thiocarbamates and/or *N*-trifluoromethyl selenocarbamates comprising the step of contacting a compound R-N(CF₃)CO-LG, whereby R is an organic moiety and LG is a leaving group, with an amine, alcohol, thiol or selenol, whereby the compound R-N(CF₃)CO-LG is prepared according to any of the claims 1 to 5.

11. The method according to any of the claims 8 or 9, whereby the compound R-N(CF₃)CO-LG is prepared according to any of the claims 1 to 5.

## Patentansprüche

1. Verfahren zur Synthese von N-Trifluormethylcarbonylverbindungen der Struktur R-N(CF₃)CO-LG, wobei R ein organischer Rest und LG eine Abgangsgruppe ist, aus Isothiocyanaten R-NCS, umfassend den Schritt
a) Kontaktieren des Isothiocyanats R-NCS mit AgF und einer elektrophilen C₁-Verbindung, wobei der Kohlenstoff im formalen Oxidationszustand (+IV) vorliegt.

2. Verfahren nach Anspruch 1, wobei die elektrophile C₁-Verbindung eine Verbindung der chemischen Struktur LG¹-CO-LG² umfasst, wobei LG¹ und LG² unabhängig voneinander Abgangsgruppen sind.

3. Verfahren nach Anspruch 1 oder 2, wobei das Verfahren in Gegenwart einer oder mehrerer zusätzlicher Fluoridverbindungen durchgeführt wird.

4. Verfahren nach einem der Ansprüche 1 bis 3, wobei das Molverhältnis von AgF zu Isothiocyanat ≥ 4 ist.

5. Verfahren nach einem der Ansprüche 1 bis 4, wobei das Molverhältnis der wirksamen elektrophilen C₁-Verbindung zu Isothiocyanat ≥ 1,0 bis ≤ 2 ist.

6. Verfahren nach einem der Ansprüche 1 bis 5, wobei das Isothiocyanat R-NCS und/oder die elektrophile C₁-Verbindung und/oder das AgF *in situ* während des Reaktionsverlaufs gebildet werden.

7. Verfahren nach einem der Ansprüche 1 bis 6, wobei AgOCF₃ als Vorläufer für AgF und die elektrophile C₁-Verbindung verwendet wird.

8. Verfahren zur Synthese von N-Trifluormethylamiden, umfassend den Schritt des In-Kontakt-Bringens einer Verbindung R-N(CF₃)CO-LG, wobei R ein organischer Rest ist und LG eine Abgangsgruppe ist, mit einer metallorganischen Verbindung.

9. Verfahren nach Anspruch 8, wobei die metallorganische Verbindung eine Organomagnesiumverbindung, Organolithiumverbindung und/oder Organozinkverbindung ist.

10. Verfahren zur Synthese von *N-*Trifluormethylharnstoffen, *N-*Trifluormethylcarbamaten, *N*-Trifluormethylthiocarbamaten und/oder *N-*Trifluormethylselenocarbamaten, umfassend den Schritt des In-Kontakt-Bringens einer Verbindung R-N(CF₃)CO-LG, wobei R eine organische Komponente ist und LG eine Abgangsgruppe ist, mit einem Amin, Alkohol, Thiol oder Selenol, wobei die Verbindung R-N(CF₃)CO-LG gemäß einem der Ansprüche 1 bis 5 hergestellt wird.

11. Verfahren nach einem der Ansprüche 8 oder 9, wobei die Verbindung R-N(CF₃)CO-LG nach einem der Ansprüche 1 bis 5 hergestellt wird.

## Revendications

1. Procédé pour la synthèse de composés de N-trifluorométhylcarbonyle ayant la structure R-N(CF₃)CO-LG, dans laquelle R est un groupement organique et LG est un groupe partant, à partir d'isothiocyanates R-NCS, comprenant l'étape de
a) mise en contact de l'isothiocyanate R-NCS avec AgF et un composé électrophile en C₁ où le carbone est à l'état d'oxydation formel (+IV).

2. Procédé selon la revendication 1, dans lequel le composé électrophile en C₁ comprend un composé de la structure chimique LG¹-CO-LG² dans laquelle LG¹ et LG² sont indépendamment les uns des autres des groupes partants.

3. Procédé selon la revendication 1 ou 2, dans lequel le procédé est mis en œuvre en présence d'un ou plusieurs composés de fluorure supplémentaires.

4. Procédé selon l'une quelconque des revendications 1 à 3, dans lequel le rapport molaire d'AgF sur l'isothiocyanate est ≥ 4.

5. Procédé selon l'une quelconque des revendications 1 à 4, dans lequel le rapport molaire du composé électrophile en C₁ efficace sur l'isothiocyanate est ≥ 1,0 à ≤ 2.

6. Procédé selon l'une quelconque des revendications 1 à 5, dans lequel l'isothiocyanate R-NCS et/ou le composé électrophile en C₁ et/ou l'AgF sont formés *in situ* au cours de la réaction.

7. Procédé selon l'une quelconque des revendications 1 à 6, dans lequel AgOCF₃ est utilisé comme précurseur pour AgF et le composé électrophile en C₁.

8. Procédé de synthèse de *N*-trifluorométhylamides comprenant l'étape de mise en contact d'un composé R-N(CF₃)CO-LG, dans lequel R est un groupement organique et LG est un groupe partant, avec un composé organométallique.

9. Procédé selon la revendication 8, dans lequel le composé organométallique est un composé organomagnésium, un composé organolithium et/ou un composé organozinc.

10. Procédé de synthèse de *N*-trifluorométhylurées, N-trifluorométhylcarbamates, *N-*trifluorométhylthiocarbamates et/ou *N-*trifluorométhylsélénocarbamates, comprenant l'étape de mise en contact d'un composé R-N(CF₃)CO-LG, dans lequel R est un groupement organique et LG est un groupe partant, avec une amine, un alcool, un thiol ou un sélénol, dans lequel le composé R-N(CF₃)CO-LG est préparé selon l'une quelconque des revendications 1 à 5.

11. Procédé selon l'une quelconque des revendications 8 ou 9, dans lequel le composé R-N(CF₃)CO-LG est préparé selon l'une quelconque des revendications 1 à 5.
